# EUROPEAN PATENT APPLICATION

(11) **EP 4 670 714 A1**
(43) Date of publication of application: **31.12.2025**
(21) Application number: 24760567.8
(22) Date of filing: 21.02.2024
(51) Int. Cl.: A61K 31/192, A61P 21/00, A61P 43/00, A61P 35/00, A23L 33/10, A23K 20/111

(54) **COMPOSITION FOR TREATING CANCER CACHEXIA, COMPRISING DIFLUNISAL OR A PHARMACEUTICALLY ACCEPTABLE SALT THEREOF**

(30) Priority: 24.02.2023 KR 20230025224
(71) Applicant: Animuscure Inc., Suwon-si, Gyeonggi-do 16419 (KR)
(72) Inventor: LEE, Sang-Jin, Seoul 04747 (KR)
(74) Representative: J A Kemp LLP
(86) International application number: PCT/KR2024/002259
(87) International publication number: WO 2024/177378

(57) **Abstract**

The present invention relates to a composition for ameliorating and treating a muscle disease and cachexia, which includes diflunisal. The diflunisal has an effect of promoting the differentiation and proliferation of myoblast cells, and also has an effect of ameliorating and treating cachexia caused by muscle diseases or chronic diseases such as cancer through effects such as increasing muscle mass, recovering muscle strength, and the like.

## Description

### [Technical Field]

The present invention relates to a composition for ameliorating and treating cachexia, particularly cancer cachexia, which includes diflunisal, which is a non-steroidal analgesic drug, or a pharmaceutically acceptable salt thereof.

### [Background Art]

Cachexia is a condition in which muscle loss occurs continuously due to various diseases such as cancer, heart failure, chronic diseases, and the like. In particular, cancer cachexia is a complex metabolic syndrome accompanying cancer. It refers to a state of systemic malnutrition in which cancer patients experience loss of body mass (body weight, muscle, and the like) that is not restored due to limited utilization or abnormal metabolism of nutrients even when nutrients are supplied through normal food intake. It is generally defined as a loss of 5% or more of body weight due to muscle and fat loss within 6 months after the onset of cancer. Although it does not occur in all cancer patients, it is reported that approximately 50% to 60% of patients with gastrointestinal, pancreatic, lung, and colon cancer have cancer cachexia.

The cause of cancer cachexia is not exactly known, but inflammatory cytokines such as IL-6, TNF-α, and C-reactive protein 1 (CRP1) are known to play a central role in the loss of muscle and fat. There is no specific method of treating cancer cachexia, but food intake may be indirectly increased by ameliorating pain or gastrointestinal disorders, or appetite stimulants may be used. In recent years, drugs such as a COX-2 inhibitor (*i.e.*, celecoxib) and a TNF-α inhibitor (*i.e.*, thalidomide) have been used, but celecoxib has been reported to cause side effects such as anemia, gastric ulcers, allergies, heart attack, stroke, and the like, and thalidomide has been reported to cause side effects such as depression, heart failure, dyspnea, vomiting, rashes, hypertension, and birth defects during pregnancy. Above all, there is insufficient evidence for a clear therapeutic effect.

The present inventors completed the invention after conducting research on a drug that has a therapeutic effect on cachexia, particularly cancer cachexia, has few side effects, and may be safely administered to patients.

### [Disclosure]

### [Technical Problem]

In order to screen drugs that may be used for the treatment of a muscle disease, particularly cachexia, among NSAIDs, the present inventors have confirmed effects associated with muscle disease and cachexia, such as increased body weight and muscle mass, improved muscle strength, and the like for various drugs, and found that diflunisal has an excellent effect of ameliorating and treating symptoms related to the muscle disease or cachexia. Therefore, the present invention has been completed based on these facts.

Therefore, the present invention is directed to providing a composition for preventing or treating a muscle disease, which includes diflunisal or a pharmaceutically acceptable salt thereof.

Also, the present invention is directed to providing a pharmaceutical composition for ameliorating or treating cachexia, which includes diflunisal or a pharmaceutically acceptable salt thereof.

In addition, the present invention is directed to providing a composition for muscle enhancement or muscle strengthening, which includes diflunisal or a pharmaceutically acceptable salt thereof.

Further, the present invention is directed to providing an anticancer adjuvant composition including diflunisal or a pharmaceutically acceptable salt thereof.

### [Technical Solution]

In order to solve the above problems, according to an aspect of the present invention, there is provided a composition for preventing or treating a muscle disease, which includes diflunisal or a pharmaceutically acceptable salt thereof.

According to another aspect of the present invention, there is provided a pharmaceutical composition for ameliorating or treating cachexia, which includes diflunisal or a pharmaceutically acceptable salt thereof.

According to still another aspect of the present invention, there is provided a composition for muscle enhancement or muscle strengthening, which includes diflunisal or a pharmaceutically acceptable salt thereof.

According to yet another aspect of the present invention, there is provided an anticancer adjuvant composition including diflunisal or a pharmaceutically acceptable salt thereof.

Hereinafter, the present invention will be described in detail.

According to one aspect of the present invention, the present invention relates to a composition for preventing or treating a muscle disease, which includes diflunisal or a pharmaceutically acceptable salt thereof.

The diflunisal is a salicylate derivative and belongs to the non-steroidal anti-inflammatory drug (NSAID) family of drugs. In particular, it has been widely used as an analgesic agent for musculoskeletal diseases, and is a compound represented by the following Chemical Formula 1.

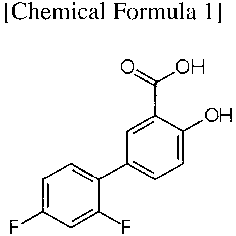

Diflunisal is a competitive COX-I and COX-II inhibitor (high affinity for COX-I), which acts by inhibiting the conversion of arachidonic acid in the body to precursors of prostaglandins that cause inflammation and fever, and is used as an anti-inflammatory and analgesic agent.

In the present invention, the diflunisal has a prophylactic, ameliorating or therapeutic effect on muscle diseases exhibiting symptoms such as muscle dysfunction, muscle loss, muscle atrophy, muscle wasting, or muscle degeneration. The "muscle disease" refers to a condition in which muscular strength is weakened due to muscle damage or loss caused by aging or diseases. In this case, the muscle disease is caused by various causes such as genetic predisposition; age-related diseases such as hypertension, impaired glucose tolerance, diabetes, obesity, dyslipidemia, atherosclerosis or cardiovascular disease; chronic diseases such as cancer, autoimmune disease, infectious disease, AIDS, chronic inflammatory disease, arthritis, malnutrition, kidney disease, chronic obstructive pulmonary disease, emphysema, rickets, chronic lower spinal pain, peripheral nerve damage, central nerve damage, and chemical damage; fractures, trauma, and the like, or loss of movement due to long-term bed rest; aging, and the like.

The "muscle disease" of the present invention may be at least one muscle disease selected from the group consisting of atony, muscular atrophy, muscular dystrophy, muscle degeneration, myotonic dystrophy, amyotrophic lateral sclerosis, myasthenia, cachexia, and senile sarcopenia, particularly, may be a disease such as a muscle disease due to senile muscular atrophy, cancer and a chronic disease, or muscle atrophy due to muscle disuse, and more specifically, may include muscular atrophy due to senile muscular atrophy or cancer, muscular dystrophy, muscle degeneration, myotonic dystrophy, amyotrophic lateral sclerosis, myasthenia, cachexia, and senile sarcopenia, but the present invention is not limited thereto. In particular, in the present invention, the muscle disease may be caused by cancer or a chronic disease.

The diflunisal of the present invention has an effect of ameliorating or treating cachexia, especially among muscle diseases. The cachexia is a severe systemic wasting syndrome that appears in the terminal stages of diseases such as cancer, tuberculosis, diabetes, and AIDS, and especially frequently appears in patients with gastrointestinal cancer and lung cancer. The main symptoms are decreased appetite, decreased weight and physical strength due to muscle and fat loss, and the like, and lead to a state in which weight is lost despite normal food intake. Cachexia may reduce the therapeutic effect of the diseases and shorten the life expectancy of the patients.

In the present invention, the cachexia to be ameliorated or treated may be cancer cachexia caused by cancer.

In the present invention, cancers that may cause cachexia may include melanoma, leukemia, lymphoma, myeloma, myelodysplastic syndrome, breast cancer, head and neck cancer, esophageal cancer, stomach cancer, colorectal cancer (=colon cancer), rectal cancer, anal cancer, hepatocellular carcinoma, cholangiocarcinoma, gallbladder cancer, pancreatic cancer, lung cancer (non-small cell lung cancer, small cell lung cancer), thymic cancer, kidney cancer, bladder cancer, prostate cancer, testicular cancer, ovarian cancer, cervical cancer, sarcoma, gastrointestinal stromal tumors, cancer of unknown primary site, mesothelioma, neuroendocrine tumors, skin cancer, blood cancer, and the like, but the present invention is not limited thereto. More preferably, the cancers may be digestive organ cancer such as stomach cancer, colon cancer, rectal cancer, liver cancer, gallbladder cancer, and pancreatic cancer, or lung cancer.

The pharmaceutical composition according to the present invention may ameliorate and treat one or more symptoms caused by cachexia selected from the group consisting of decreased appetite, weight loss, increased fatigue, muscle weakness, decreased motor activity, muscle loss, fat loss, hematopoietic toxicity, and the like, and preferably may ameliorate and treat weight loss, muscle loss, muscle weakness, or decreased motor activity.

In relation to the above-described effects, in one embodiment of the present invention, when diflunisal was administered to a cancer cachexia-induced model, it was confirmed that diflunisal had effects of increasing body weight and muscle mass and enhancing muscle strength, and that the weight loss of organs such as the spleen, liver, heart, and the like was improved, compared to the non-administered group, in the cachexia-induced model. This shows that diflunisal may be used to ameliorate and treat cancer cachexia.

According to another aspect of the present invention, the diflunisal may be used as a composition having a muscle enhancement, muscle strengthening, or exercise performance enhancement effect.

In one embodiment of the present invention, it was confirmed that the diflunisal has an effect of promoting the proliferation and differentiation of muscle stem cells. In this embodiment, diflunisal increased the expression of Pax7, which is a muscle stem cell proliferation marker, and increased the expression levels of MHC and myogenin, which are muscle stem cell differentiation markers. Also, it was confirmed that the diameter of muscle fibers increased when diflunisal was administered. That is, diflunisal may promote the proliferation and differentiation of muscle stem cells, and thus may have an effect of improving muscle function such as increasing muscle mass due to an increase in muscle mass, improving muscular strength, enhancing muscle recovery, and reducing muscle fatigue. The above-described effect was confirmed equally not only in the disease (cancer cachexia)-induced model but also in normal muscles.

Also, the diflunisal may improve exercise performance ability. The term "exercise performance ability" refers to the ability to perform exercise using muscular strength. At this time, the muscular strength may be improved by increasing muscle mass, muscle endurance, oxidative muscle mass, improving muscle recovery ability and intramuscular energy balance, and may also be enhanced by reducing fatigue substances within the muscle. In one embodiment of the present invention, the effect of improving muscle endurance and increasing muscle mass in mice was confirmed. Based on these results, it can be seen that the exercise performance ability may be improved.

The composition of the present invention may be used for various purposes, such as medical supplies, health functional foods, functional foods, animal feed, cell culture compositions, and the like, and may have prophylactic, ameliorating or therapeutic effects on muscle diseases and cachexia, according to the effects of promoting the proliferation and differentiation of muscle stem cells, increasing muscle mass, and improving muscle endurance.

In the present specification, the term "prevention" refers to any action that may suppress or delay the onset of a disease by administering the pharmaceutical composition according to the present invention.

In the present specification, the term "amelioration" refers to any action that may alleviate or delay the onset of symptoms caused by a disease by administering the pharmaceutical composition according to the present invention.

In the present specification, the term "treatment" refers to any action that improves or beneficially changes the symptoms by administering the pharmaceutical composition according to the present invention.

The pharmaceutical composition of the present invention may be formulated in the form of oral formulations such as powders, granules, tablets, capsules, suspensions, emulsions, syrups, aerosols, and the like, external preparations, suppositories, and sterile injection solutions according to conventional methods, and may further include carriers or excipients necessary for the formulation. Pharmaceutically acceptable carriers, excipients, and diluents that may be included along with the active ingredient include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, magnesium stearate, mineral oil, and the like. When formulated, the pharmaceutical composition is prepared using commonly used diluents or excipients such as fillers, bulking agents, binders, wetting agents, disintegrants, surfactants, and the like.

For example, solid preparations for oral administration include tablets, pills, powders, granules, capsules, and the like. These solid preparations are prepared by mixing an extract or compound with at least one excipient, at least cotton, starch, calcium carbonate, sucrose or lactose, gelatin, and the like. In addition to simple excipients, lubricants such as magnesium stearate and talc are also used. Liquid preparations for oral administration include suspensions, oral solutions, emulsions, syrups, and the like. In addition to commonly used simple diluents such as water and liquid paraffin, the liquid preparations for oral administration may include various excipients such as wetting agents, sweeteners, fragrances, preservatives, and the like.

Preparations for parenteral administration include sterile aqueous solutions, non-aqueous solutions, suspensions, emulsions, lyophilized preparations, and suppositories. Propylene glycol, polyethylene glycol, vegetable oils such as olive oil, injectable esters such as ethyl oleate, and the like may be used as the non-aqueous solutions and suspensions. Witepsol, macrogol, Tween 61, cacao butter, laurin butter, glycerogelatin, and the like may be used as a base for suppositories.

The pharmaceutical composition of the present invention may be administered orally or parenterally (applied intravenously, subcutaneously, intraperitoneally, or topically) depending on the intended method. The dose of the pharmaceutical composition may vary depending on the patient's condition and weight, the severity of a disease, the drug form, a route of administration, and the administration time, and may be selected in an appropriate form by a person having skill in the art.

The pharmaceutical composition of the present invention is administered in a pharmaceutically effective amount. In the present invention, the term "pharmaceutically effective amount" refers to a reasonable amount applicable to medical treatment, that is, an amount sufficient to treat a disease, and a level of the effective amount may be determined according to the type of a patient's disease, the severity of the disease, the activity of a drug, the sensitivity to the drug, the time of administration, the route of administration, and the excretion rate, the duration of treatment, concomitantly used ingredients, and other factors. The pharmaceutical composition according to the present invention may be administered as an individual therapeutic agent or in combination with other therapeutic agents, and may be administered sequentially or concurrently with conventional therapeutic agents. The dose may be determined at a level that may minimize side effects in consideration of all of the above factors, and may be easily determined by a person having skill in the art. Specifically, the dose of the pharmaceutical composition may vary depending on the patient's age, weight, severity, sex, and the like. In this case, the pharmaceutical composition may be generally administered daily, every other day, or 1 to 3 times a day at a dose of 0.001 to 150 mg, more preferably 0.01 to 100 mg per 1 kg of body weight. However, the above dose is exemplary and may be set differently when necessary.

Also, the composition of the present invention may be a food or a health functional food. In particular, the term "health functional food" refers to a food manufactured and processed using raw materials or ingredients having functionality useful to the human body according to Act No. 6727 on Health Functional Foods, and the term "functionality" means that the food is taken for the purpose of regulating nutrients for the structure and function of the human body or obtaining useful effects for health purposes such as physiological effects, and the like.

The food or health functional food of the present invention may be manufactured and processed into pharmaceutical dosage forms such as powders, granules, tablets, capsules, pills, suspensions, emulsions, syrups, and the like, or health functional foods such as tea bags, infusions, beverages, candies, jellies, gums, and the like for the purpose of preventing and ameliorating a muscle disease.

The food or health functional food composition of the present invention may be used as a food additive, and may be manufactured into products alone or in combination with other ingredients. Also, the food or health functional food composition may include nutrients, vitamins, electrolytes, flavoring agents, coloring and enhancing agents, pectic acid and salts thereof, alginic acid and salts thereof, organic acids, protective colloid thickeners, pH regulators, stabilizers, preservatives, glycerin, alcohol, carbonating agents used in carbonated beverages, and the like. The ingredients may be used alone or in combination, and may be combined and used in an appropriate amount.

According to another aspect of the present invention, the present invention relates to an anticancer adjuvant composition including diflunisal. The anticancer adjuvant composition may be used as an adjuvant for cancer treatment using a known anticancer agent, and may be administered simultaneously or sequentially with the anticancer drug as an anticancer adjuvant.

### [Advantageous Effects]

The present invention relates to a composition having an effect of ameliorating and treating cachexia, particularly cancer cachexia, which includes diflunisal or a pharmaceutically acceptable salt thereof. The composition is intended for use in ameliorating various symptoms accompanying cachexia, such as weight loss, and for use in treating cachexia by increasing muscle mass, improving muscle endurance, and increasing motor activity through promoting proliferation and differentiation of myoblast cells.

### [Description of Drawings]

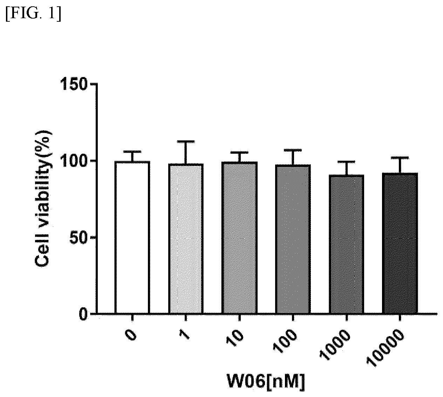

FIG. 1 shows the results of evaluating the cytotoxicity of diflunisal (MTT assay) using C₂C₁₂ cells, which are muscle stem cells.

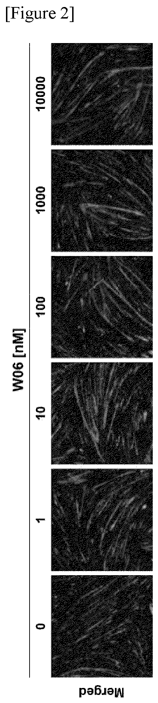

FIG. 2 shows the results of MHC fluorescence staining, showing that the muscle cell differentiation-promoting effect of diflunisal is confirmed *in vitro.*

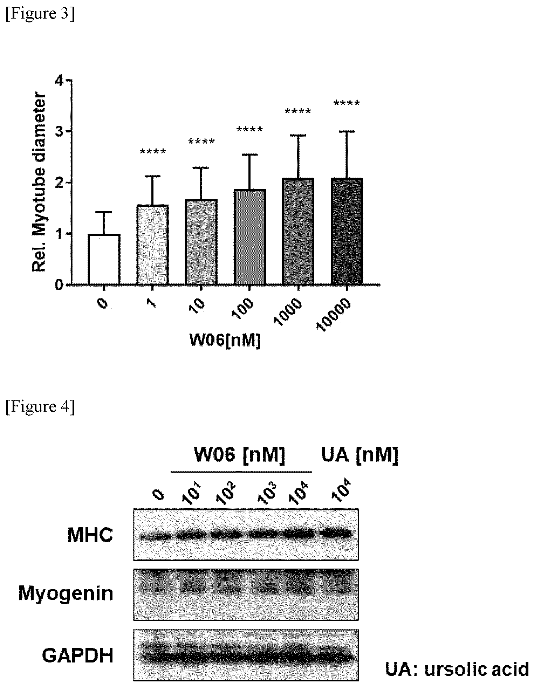

FIG. 3 shows the results of confirming the muscle cell differentiation-promoting effect of diflunisal *in vitro,* in which results are compared by quantifying the diameter of the myotubes.
FIG. 4 shows the results of immunoblot analysis for MHC and myogenin, showing that the muscle cell differentiation-promoting effect of diflunisal is confirmed *in vitro.*

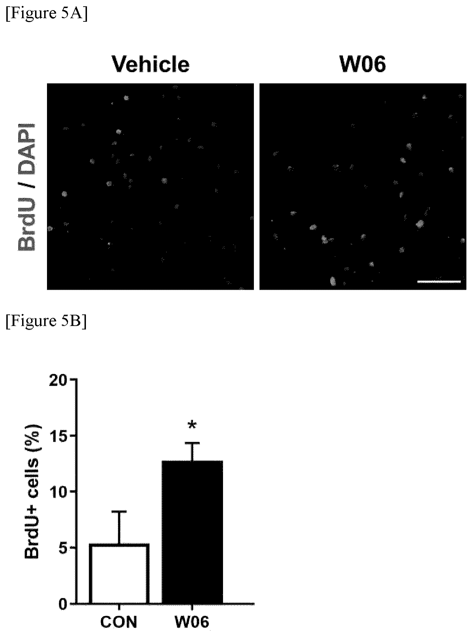

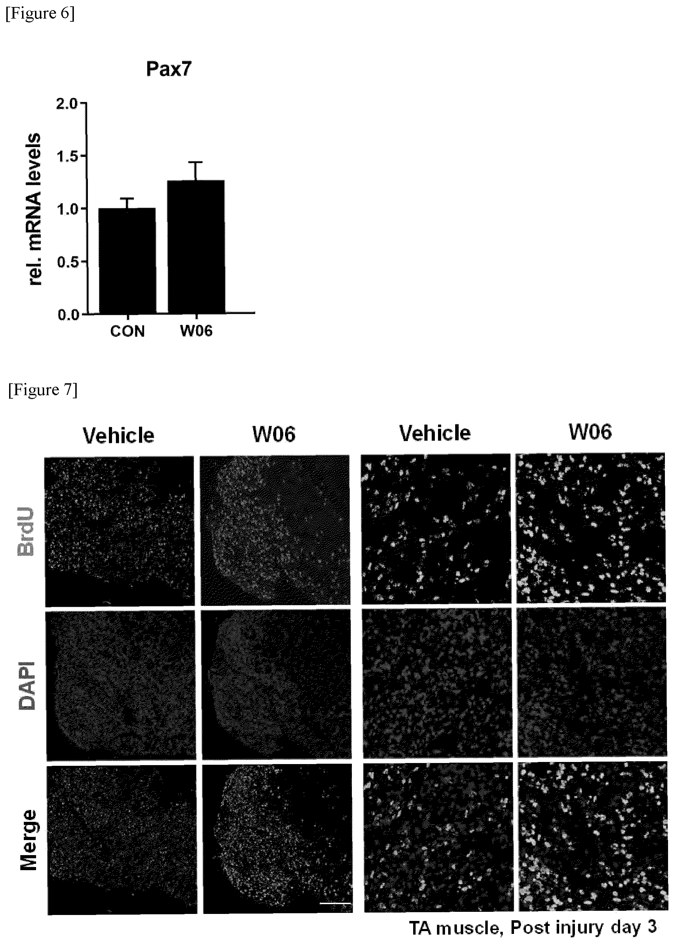

FIGS. 5 and 6 show the results of confirming the stem cell differentiation-promoting effect through administration of diflunisal. FIG. 5 shows the results of BrdU staining of C₂C₁₂ muscle stem cells and quantification of BrdU-positive (BrdU+) cells, and FIG. 6 shows the results of qRT-PCR analysis for the expression of Pax7, which is a stem cell marker.

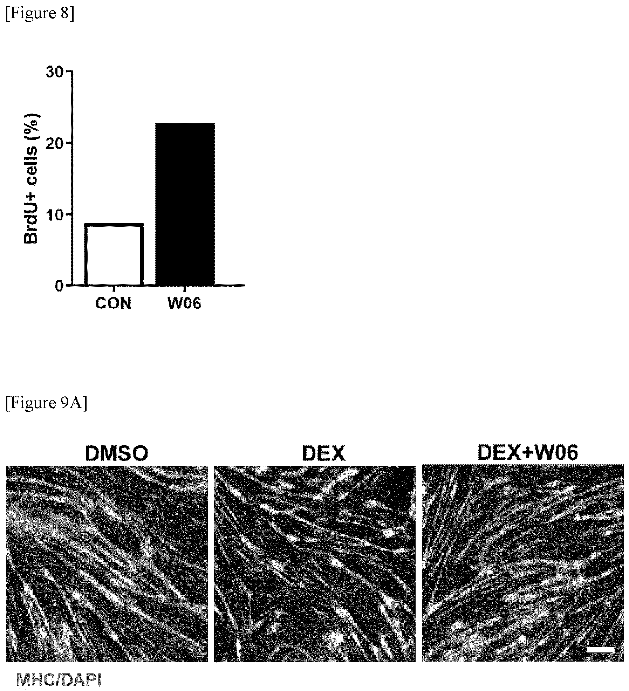

FIGS. 7 and 8 show the muscle stem cell proliferation-promoting effect through administration of diflunisal in the tibialis anterior (TA) muscle of mice with muscle damage induced by CTX. FIG. 7 shows a BrdU staining image, and FIG. 8 shows the results of quantification of BrdU-positive (BrdU+) cells.

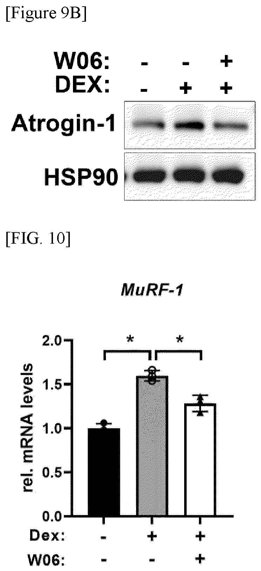

FIGS. 9 and 10 show the effect of ameliorating muscular atrophy when diflunisal is adminstered to a model in which muscle atrophy is induced by administration of dexamethasone. FIGS. 9A and 9B show the results of MHC immunostaining and the results of immunostaining analysis for atrogin-1 expression, respectively, and FIG. 10 shows the results of qRT-PCR analysis for MuRF1 expression.

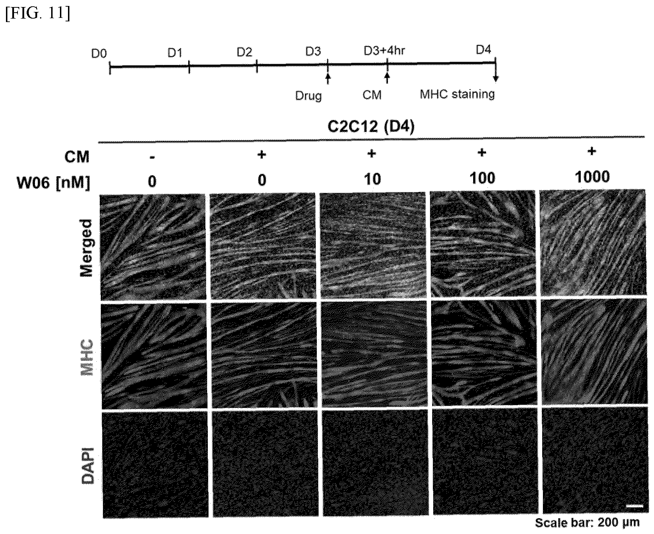

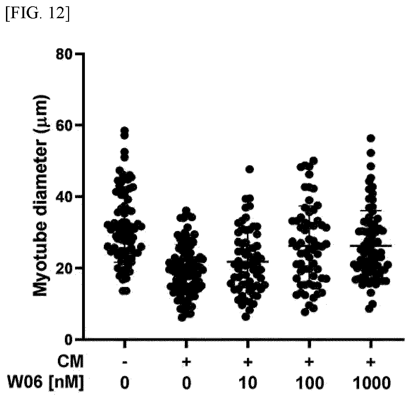

FIGS. 11 and 12 show the results of confirming the *in vitro* amelioration effect of diflunisal administration on cachexia induced by a cancer cell culture medium (CM). FIG. 11 shows the results of confirming the concentration-dependent myotube regeneration and amelioration effect of diflunisal administration in a cancer cachexia-induced model using MHC immunostaining, and FIG 12 shows the results of quantification by measuring the diameter of myotubes.

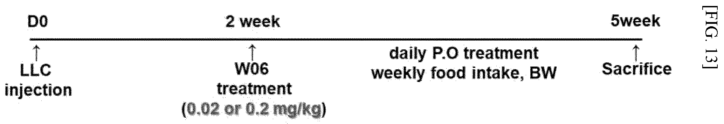

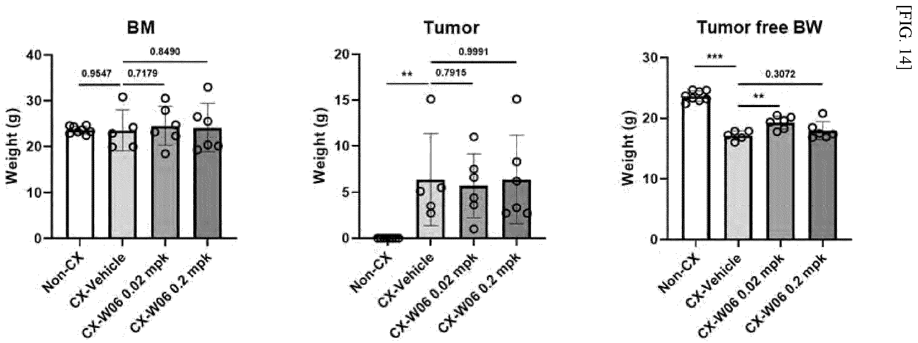

FIGS. 13 and 14 show the results of confirming the effect of diflunisal administration by inducing cancer cachexia *in vivo.* FIG. 13 shows a schematic diagram of an *in vivo* experimental method, and FIG. 14 shows the results of measuring the changes in body weight, tumor weight, and tumor-free body weight after administration of diflunisal.

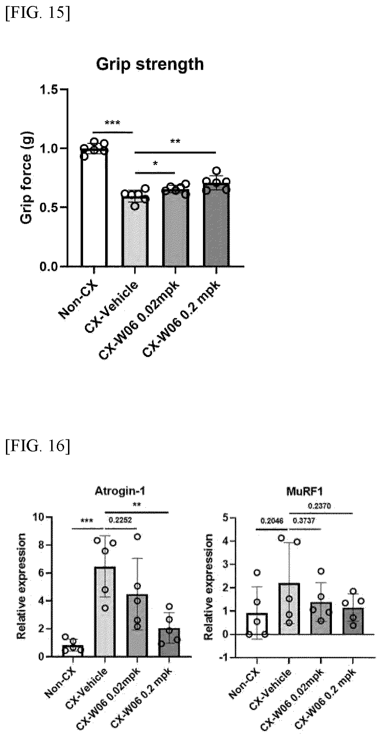

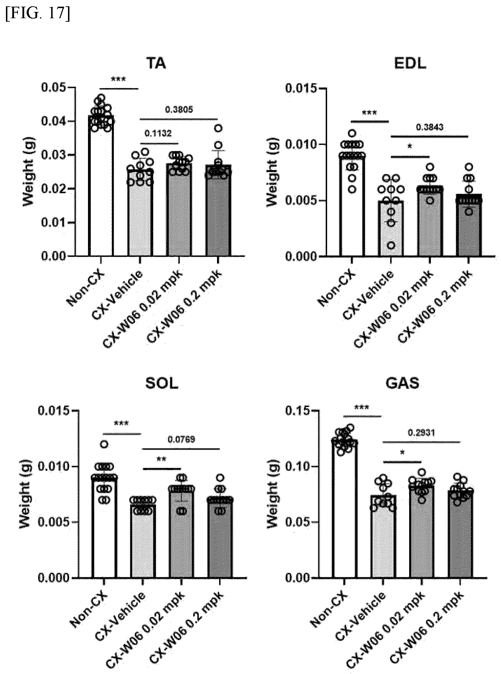

FIGS. 15 to 17 show the results of examining the effects of diflunisal administration on cancer cachexia induced *in vivo*, including the results of examining motor ability (grip strength) (FIG. 15), the results of Atrogin-1 and MuRF1 mRNA expression analysis (qRT-PCR) (FIG. 16), and the results of measuring muscle mass in each part of the hind limb (FIG. 17), respectively.

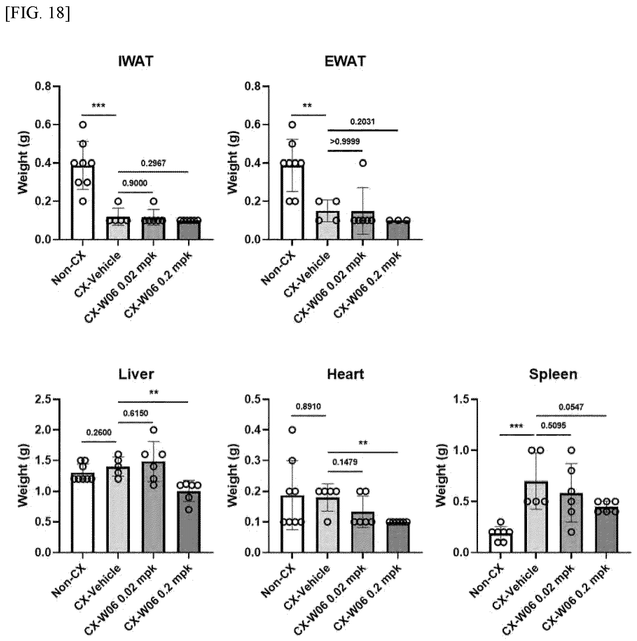

FIG. 18 shows the amelioration effect of diflunisal on cancer cachexia induced *in vivo*, including changes in the weights of the inguinal white adipose tissue (IWAT), visceral adipose tissue (EWAT), liver, heart, and spleen.

### [Best Mode]

Hereinafter, examples will be described in detail to specifically explain the present specification. However, it should be understood that the examples according to the present specification may be modified into various different forms, and the scope of the present specification is not intended to be limited to the examples described below. The examples of the present specification are provided to more completely explain the present specification to a person having ordinary skill in the art.

### Example 1: Myoblast cell line culture and differentiation induction

C₂C₁₂ is a myoblast cell line obtained from the C3H mouse strain, and is widely used to study muscle cell differentiation. The cells were cultured in a cell culture medium (growth medium (GM)), and muscle cell differentiation was induced in a differentiation medium (DM). DMEM containing 15% fetal bovine serum (FBS) was used as the cell culture medium, and DMEM containing 2% horse serum was used as the differentiation medium.

To induce muscle cell differentiation, cells were seeded in a cell culture medium and cultured. When the density of cells reached approximately 80% to 90%, the medium was replaced with a differentiation medium, and differentiation was induced for approximately 2 to 3 days.

### Experimental Example 1: Evaluation of cytotoxicity of diflunisal

The cytotoxicity of diflunisal (CAS No. 22494-42-4, W06) was evaluated using an MTT assay. C₂C₁₂ myoblast cells were treated with diflunisal at concentrations of 0, 1, 10, 100, 1,000, and 10,000 nM for 18 hours, and cell viability was then confirmed using the MTT assay. Myoblast cells (1.0 X 10⁴ cells /96 well) were allowed to react with 5 mg/mL MTT for 4 hours in a cell culture medium, and the eluted reaction product was then measured for absorbance at 570 nm. As a result, it was confirmed that when the cells were treated with approximately 10,000 nM of diflunisal used in the MTT assay, cell viability was almost similar to that of the control (FIG. 1).

### Experimental Example 2: Confirmation of stem cell differentiation-promoting effect of diflunisal

To analyze the effect of diflunisal on the differentiation of muscle stem cells, MHC immunofluorescence staining was performed (FIG. 2). C₂C₁₂ cell lines were treated with the control DMSO and diflunisal at concentrations of 10, 100, 1,000, and 10,000 nM, respectively, to induce muscle cell differentiation. On day 3 of differentiation induction, immunofluorescence staining was performed using an MHC antibody to compare and analyze the degree of myotube formation. As a result, muscle cell differentiation was promoted in a concentration-dependent manner in the cells treated with diflunisal compared to the control. Also, the results of quantifying the diameter of myotubes also confirmed that muscle cell differentiation increased in a concentration-dependent manner when the cells were treated with diflunisal (FIG. 3).

In addition, the expression levels of differentiation markers were confirmed through immunoblotting. It was confirmed that diflunisal treatment induced an increase in the expression of muscle stem cell differentiation markers MHC and myogenin, indicating that the expression of MHC and myogenin was significant compared to when the cells were treated with ursolic acid (UA), which is a positive control (FIG. 4).

### Experimental Example 3: Confirmation of stem cell proliferation-promoting effect of diflunisal

To analyze the effect of diflunisal on the proliferation of myoblast cells, a BrdU staining experiment was performed. C₂C₁₂ myoblast cells were treated with DMSO as a control or diflunisal (1 µM) for approximately 20 hours, and then allowed to react with BrdU for 15 minutes. Thereafter, immunofluorescence staining was performed using an antibody against BrdU (FIG. 5A). As a result, it was confirmed that the proliferation of myoblast cells increased in the group treated with diflunisal. Also, a quantitative comparison confirmed that when the cells were treated with diflunisal, the proportion of BrdU-positive cells significantly increased compared to the control (FIG. 5B).

In addition, it was confirmed whether the expression level of Pax7, which is a muscle proliferation marker, increased due to diflunisal treatment. As a result, as shown in FIG. 6, it was confirmed that the relative expression level of Pax7 mRNA increased when the cells were treated with diflunisal.

### Experimental Example 4: Stem cell proliferation-promoting effect in animal model of muscle damage

This experiment was conducted to determine whether diflunisal had an effect of promoting the proliferation of stem cells when diflunisal was administered even in an animal model in which actual muscle damage had occurred. C57BL/6 male mice were orally administered diflunisal at a dose of 0.02 mg/kg for 2 weeks, and then on the second week, 10 µM cardiotoxin (CTX) was directly injected into the muscle at 2 µL per g of body weight to induce muscle damage. Thereafter, diflunisal was administered at a dose of 0.02 mg/kg for 3 days. Then, immunofluorescence staining was performed on the TA muscle tissue of the mice using a BrdU antibody. As a result, as shown in FIG. 7, it was confirmed that the proliferation of myoblast cells increased in the group treated with diflunisal. Also, a quantitative comparison confirmed that the proportion of BrdU-positive cells increased by more than 2.5 times compared to the control when the cells were treated with diflunisal (FIG. 8).

### Experimental Example 5: Muscle atrophy-ameliorating effect

To determine whether diflunisal could recover or protect muscles from dexamethasone (DEX)-induced muscle atrophy, this experiment was performed using C₂C₁₂ myoblast cells. C₂C₁₂ myoblast cells were differentiated in a differentiation medium (DM) for 2 days, pretreated with 100 µM DEX for 4 hours, and then treated with diflunisal (1 µM) together with DMSO as a vehicle. Then, the C₂C₁₂ myoblast cells were further cultured in DM for 24 hours.

Immunofluorescence staining was performed on dexamethasone-treated C₂C₁₂ myotube cells using an MHC antibody to compare and analyze the degree of myotube formation. As a result, as shown in FIG. 9, it can be seen that when muscle atrophy was induced by dexamethasone, MHC expression decreased, whereas when diflunisal was administered, MHC expression was restored. Also, when the expression level of atrogin-1, which is a muscle atrophy marker, was confirmed through immunoblotting, it was confirmed that its expression increased when dexamethasone was administered. However, it was found that its expression decreased again when diflunisal was administered (FIG. 9).

Also, from the results of qRT-PCR analysis of the expression of MuRF1, which is another muscle atrophy marker, it was confirmed that MuRF1 expression increased when muscle atrophy was induced, but its expression was significantly reduced when diflunisal was administered (FIG. 10).

### Experimental Example 6: Amelioration effect on cachexia induced by cancer cell culture medium

To confirm the amelioration effect of diflunisal on cancer cachexia, cancer cachexia was induced in muscle cells using a cancer cell culture medium (CM) (FIG. 11, top).

In the cancer cachexia-induced model, the cells were treated with diflunisal at concentrations of 10, 100, and 1,000 nM, respectively, to confirm the myotube regeneration and improvement effects through MHC immunostaining. Also, the diameter of the myotubes was measured to confirm the improvement. As a result, as shown in FIGS. 11 and 12, it was confirmed that when the cells were cultured in a CM culture medium, cancer cachexia was induced, and thus the diameter of myotubes decreased. However, it can be seen that when diflunisal was administered, the diameter of myotubes increased in a concentration-dependent manner. This indicates that diflunisal has the effect of ameliorating muscle loss and muscle contraction caused by cancer cachexia.

### Experimental Example 7: Amelioration effect on cancer cachexia in animal model

### 7-1) Effect of improving weight loss due to cancer cachexia

Lewis lung carcinoma (LCC, 5 × 10⁶ cells/mice) was injected into C57BL/6 mice to induce lung cancer. Two weeks after injection, diflunisal was orally administered daily for 21 days at a dose of 0.02 or 0.2 mpk (FIG. 13). Thereafter, the changes in body weight, tumor weight, and tumor-free body weight for each mouse were measured. As a result, as shown in FIG. 14, it can be seen that there was no significant change in the overall body weight in all experimental groups, but the tumor weight increased in the cancer-induced group, and the change (decrease) in tumor-free body weight was improved in the diflunisal-administered group. In particular, the tumor-free body weight in the 0.02 mpk diflunisal-administered group significantly increased compared to the cancer-induced group (FIG. 14).

### 7-2) Effect of improving motor ability and muscle mass due to cancer cachexia

To compare the motor ability in the cancer cachexia-induced mouse model, a grip strength test was conducted for each experimental group. As a result, as shown in FIG. 15, it was confirmed that the grip strength of the diflunisal-administered group increased in a concentration-dependent manner compared to the cancer-induced group, indicating that the motor ability significantly increased.

Also, the expression levels of atrogin-1 and MuRF-1, which are markers associated with amyotrophy, were confirmed through immunoblotting. As a result, it was confirmed that the relative mRNA expression levels increased when cancer was induced, but their expression levels decreased again when diflunisal was administered (FIG. 16).

In addition, changes in muscle mass of the hindlimb muscles, tibialis anterior (TA), extensor digitorum longus (EDL), soleus (SOL), and gastrocnemius (GA), were confirmed as shown in FIG. 17. It was confirmed that the muscle mass of TA, EDL, SOL, and GAS all decreased when cancer was induced, and the muscle mass loss caused by cancer cachexia was significantly improved when diflunisal was administered (FIG. 17). This confirmed the effect of suppressing muscle loss caused by cancer cachexia when diflunisal was administered (FIG. 17).

### 7-3) Effect of improving weight loss due to cancer cachexia

Further, to confirm the effects of diflunisal on other parts of the body, such as white fat, visceral fat, the liver, heart, spleen, and the like, in a cancer cachexia-induced mouse model, the weights for each tissue were measured and compared. As a result, as shown in FIG. 18, it was confirmed that all the weights were reduced in the cancer-induced model, but the reduction in fat and organ weights caused by cancer cachexia was improved when diflunisal was administered.

Hereinabove, the present invention has been described and shown with reference to the preferred embodiments thereof. It will be apparent to those skilled in the art to which the present invention pertains that the present invention may be implemented in modified forms without departing from the scope of the present invention. Therefore, the disclosed embodiments are to be considered in all respects as illustrative and not restrictive. The scope of the invention is shown in the claims rather than in the foregoing description, and all differences within the scope equivalent to the above-described embodiments should be construed as being included in the present invention.

### [Mode for Invention]

According to one aspect, the present invention relates to a pharmaceutical composition for ameliorating or treating a muscle disease, which includes diflunisal or a pharmaceutically acceptable salt thereof.

The muscle disease may be selected from the group consisting of atony, muscular atrophy, muscular dystrophy, muscle degeneration, myotonic dystrophy, amyotrophic lateral sclerosis, myasthenia, and sarcopenia.

The muscle disease may be caused by aging, muscle dysfunction, muscle wasting, muscle degeneration, disuse, or muscle damage.

According to another aspect, the present invention relates to a pharmaceutical composition for ameliorating or treating cachexia, which includes diflunisal or a pharmaceutically acceptable salt thereof.

The cachexia may be cancer cachexia caused by cancer.

Also, the composition may ameliorate and treat one or more symptoms caused by cachexia selected from the group consisting of decreased appetite, weight loss, increased fatigue, muscular weakness, decreased motor activity, muscle loss, fat loss, hematopoietic toxicity, and the like.

According to still another aspect of the present invention, the present invention relates to a composition for muscle enhancement or muscle strengthening, which includes diflunisal or a pharmaceutically acceptable salt thereof.

The composition may have an effect of promoting proliferation or differentiation of muscle stem cells.

Also, the composition may be selected from the group consisting of a pharmaceutical composition, a health functional food composition, a functional food composition, or an animal feed composition.

According to yet another aspect of the present invention, the present invention relates to an anticancer adjuvant composition including diflunisal or a pharmaceutically acceptable salt thereof.

The composition may be administered in combination with one or more anticancer drugs.

According to yet another aspect of the present invention, the present invention relates to a use of diflunisal or an acceptable salt thereof for the treatment of a muscle disease.

The muscle disease may be selected from the group consisting of cachexia, atony, muscular atrophy, muscular dystrophy, muscle degeneration, myotonic dystrophy, amyotrophic lateral sclerosis, myasthenia, and sarcopenia.

Also, the muscle disease may be caused by aging, muscle dysfunction, muscle wasting, muscle degeneration, disuse, or muscle damage.

According to yet another aspect of the present invention, the present invention relates to a method of treating cachexia, which includes administering an effective amount of diflunisal or a pharmaceutically acceptable salt thereof to a subject.

Also, in the method, the diflunisal or a pharmaceutically acceptable salt thereof may be administered to the subject together with one or more anticancer drugs.

## Claims

1. A pharmaceutical composition for ameliorating or treating a muscle disease, comprising diflunisal or a pharmaceutically acceptable salt thereof.

2. The pharmaceutical composition of claim 1, wherein the muscle disease is selected from the group consisting of atony, muscular atrophy, muscular dystrophy, muscle degeneration, myotonic dystrophy, amyotrophic lateral sclerosis, myasthenia, and sarcopenia.

3. The pharmaceutical composition of claim 1, wherein the muscle disease is caused by aging, muscle dysfunction, muscle wasting, muscle degeneration, disuse, or muscle damage.

4. A pharmaceutical composition for ameliorating or treating cachexia, comprising diflunisal or a pharmaceutically acceptable salt thereof.

5. The pharmaceutical composition of claim 4, wherein the cachexia is cancer cachexia caused by cancer.

6. The pharmaceutical composition of claim 4, wherein the composition ameliorates and treats one or more symptoms caused by cachexia selected from the group consisting of decreased appetite, weight loss, increased fatigue, muscular weakness, decreased motor activity, muscle loss, fat loss, hematopoietic toxicity, and the like.

7. A composition for muscle enhancement or muscle strengthening comprising diflunisal or an acceptable salt thereof.

8. The composition of claim 7, wherein the composition has an effect of promoting proliferation or differentiation of muscle stem cells.

9. The composition of claim 7, wherein the composition is one or more selected from the group consisting of a pharmaceutical composition, a health functional food composition, a functional food composition, or an animal feed composition.

10. An anticancer adjuvant composition comprising diflunisal or a pharmaceutically acceptable salt thereof.

11. The anticancer adjuvant composition of claim 10, wherein the composition is administered in combination with one or more anticancer drugs.

12. A use of diflunisal or an acceptable salt thereof for the treatment of a muscle disease.

13. The use of claim 12, wherein the muscle disease is selected from the group consisting of cachexia, atony, muscular atrophy, muscular dystrophy, muscle degeneration, myotonic dystrophy, amyotrophic lateral sclerosis, myasthenia, and sarcopenia.

14. The use of claim 12, wherein the muscle disease is caused by aging, muscle dysfunction, muscle wasting, muscle degeneration, disuse, or muscle damage.

15. A method of treating cachexia, comprising:
administering an effective amount of diflunisal or a pharmaceutically acceptable salt thereof to a subject.

16. The method of claim 15, wherein the diflunisal or a pharmaceutically acceptable salt thereof is administered to the subject together with one or more anticancer drugs.
